(19) Europäisches Patentamt
European Patent Office
Office européen des brevets

(11) Publication number: **0 453 189 A1**

(12) **EUROPEAN PATENT APPLICATION**

(21) Application number: **91303248.8**

(22) Date of filing: **12.04.91**

(51) Int. Cl.[5]: **C07D 261/04,** C07D 417/06

(30) Priority: **17.04.90 GB 9008620**

(43) Date of publication of application: **23.10.91 Bulletin 91/43**

(84) Designated Contracting States: **AT BE CH DE DK ES FR GB GR IT LI LU NL SE**

(71) Applicant: **BP Chemicals Limited
Belgrave House 76 Buckingham Palace Road
London, SW1W 0SU (GB)**

(72) Inventor: **von Oppolzer, Wolfgang W.R.E.J.
14 Chemin de la Troupe
CH-1253 Vandouevres (CH)**

(74) Representative: **Barlow, Michael Thomas et al
BP INTERNATIONAL LIMITED Patents & Agreements Division Chertsey Road
Sunbury-on-Thames Middlesex TW16 7LN (GB)**

(54) **Process for preparing isoxazolines.**

(57) Chiral oxazolines having the general formula :

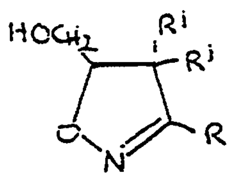

wherein R is $C_1$ to $C_{10}$ alkyl or phenyl and the $R^1$ groups are independently hydrogen or $C_1$ to $C_4$ alkyl are prepared in substatially optically pure form by a process comprising the steps of (1) reacting a nitrile oxide of formula R-C=N-O with an acryloyl derivative of formula $Z\text{-}COCH{=}C(R^1)_2$ where Z is a moiety derived from a chiral sultam and

(2) reacting the product of step (1) with a hydrogenating agent to produce the oxazoline and a chiral sultam of formula ZH.

The chiral oxalines described are useful as intermediates in e.g. the pharmaceutical or agrochemical industries.

EP 0 453 189 A1

The present invention relates to the preparation of enaniomerically pure chiral oxazolines from nitrile oxides or sources thereof.

Tetrahedron 1988 29 3555 discloses that a range of nitrile oxides can be converted into enantiomerically pure oxazolines by a process which involves as a key step reacting the nitrile oxide with a single enantiomer of an N-acryloyl substituted chiral bornane-10,2 - sultam.

According to the present invention there is provided a process for preparing a chiral oxazoline having the general formula:

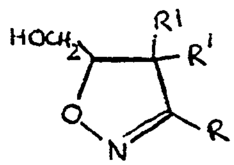

(I)

wherein R is $C_1$ to $C_{10}$ alkyl or phenyl and the $R^1$ groups are independently hydrogen or $C_1$ to $C_4$ alkyl, in substantially optically pure form, which process comprises the steps of:

(1) reacting a nitrile oxide of formula

$$R-C\equiv\overset{+}{N}-\overset{-}{O}$$

with a single enantiomer of an acryloyl derivative of formula $Z-COCH=C(R^1)_2$ wherein Z is a moiety derived from the chiral sultam defined below, and

(2) reacting the product of step (1) with a hydrogenating agent to produce the oxazoline and the chiral sultam of formula ZH.

By the term substantially optically pure form is meant an optical purity of at least 95 mole % preferably in excess of 98%.

As regards the moiety Z, this has the general formula:

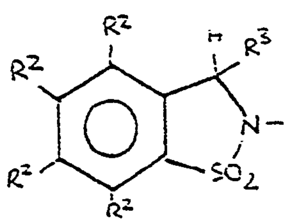

(II)

wherein the $R^2$ groups ae independently hydrogen or $C_1$ to $C_{10}$ alkyl and the $R^3$ is alkyl or aryl substituted alkyl preferably $C_1$ to $C_6$ alkyl. Most preferred $R^3$ groups are secondary or tertiary $C_3$ to $C_6$ alkyl groups eg tert-butyl.

In step (1) of the process of the present invention, the acryloyl derivative of formula $Z-COCH=C(R^1)_2$ is treated with a source of a nitrile oxide of formula

$$R-C\equiv\overset{+}{N}-\overset{-}{O}.$$

The reaction is suitably carried out at or about room temperature in a non-reactive solvent such as toluene, xylene or the like. In a preferred embodiment, the nitrile oxide, which is a highly reactive species, is generated in situ in the reaction medium by slow dehydrohalogenation of the corresponding chloroxime. The chloroxime itself can in turn be prepared by chlorination of the corresponding oxime with, for example, a chlorinating agent such as N-chlorosuccinimide. Preferred examples of nitrile oxides include those where R is selected from the group consisting of methyl, ethyl, tert-butyl or phenyl.

The product of step (1) which has the formula:

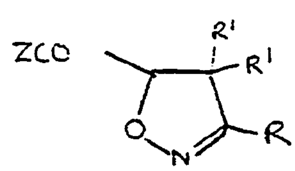

can either be isolated using standard techniques if desired.

In step (2) of the process of the present invention, the product of step (1) is treated with a hydrogenating agent to generated the oxazoline and liberate a chiral sultam of formula ZH. This reduction is suitable carried out at or about room temperature using a metal hydride or derivative thereof. Preferred hydrogenating agents are the borohydride derivatives of formula $M^{+}B(X)_3H^{-}$ where M is an alkali metal and the X groups are independently primary or secondary $C_1$ to $C_{10}$ alkyl. A particularily preferred group of hydrogenating agents are the salts of the tri-sec-butylborohydride or trisiamylborohydride anions which are sold under the registered trade mark 'selectride'.

After reduction the oxazoline and chiral sultam can be recovered and purified by conventional means.

Since the chiral sultam of formula ZH can be converted into the corresponding acryloyl derivative Z-$COCH=C(R^1)_2$ by simple acylation with an acryloyl halide of formula $YCOCH=C(R^1)_2$ (Y = C1 or Br), it will be readily apparent that the process described above can be made essentially catalytic in the chiral sultam ZH and hence that, starting from two prochiral precursors

$$R-C\equiv\overset{+}{N}-\overset{-}{O}$$

and $YCOCH = C(R^1)_2$, an asymmetric synthesis of a chiral oxazoline in optically pure form can been achieved.

The oxazolines of the present invention can serve as precursors for the syntheses of enantio- and diastereo-merically pure 1,3 -amino - alcohols and other products important for the preparation of pharmaceuticals, agrochemicals and flavour/fragrance compounds.

The invention is now illustrated by the following Examples.

Example 1

Preparation of the Acryloyl Derivative-N-[Acryloyl] -(3R)-tert-butyl-2,3-dihydro-1,2-benzisothiazole-1, 1-dioxide

The acryloyl derivative mentioned above was prepared in a three step synthesis from sacharine using the following procedure.

A. Synthesis of 3-tert-butyl-1,2-benziosothiazole-1,1-dioxide

To a solution of sacharine (12.5g, 0.066 moles) in THF (500 ml) at -78°C was slowly added a solution of tert-butyllithium (1.4 M in pentane, 100 ml, 0.14 mol). After 1.5 h the reaction was carefully quenched with $H_2O$ (20 ml). The THF was then evaporated and the residue triturated with EtOAc (300 ml). The organic phase was washed succesively with $H_2O$, 1N HC1 and brine. Drying ($MgSO_4$), decolourisation with charcoal and evaporation furnished the crude product. Recrystallisation from EtOAc furnished the pure title compound (10 g, 67% M.p.:129-130).

B. Synthesis of (3R)-tert-butyl-2,3-dihydro-1,2 benzisothiazole-1,1-dioxide

To a solution of $LiAlH_4$ in $Et_2O$ (27 ml, 1 M 27.3 mmol) at r.t. was slowly added (+)-N-methyl-ephedrine (5 g, 27.94 mmol) in $Et_2O$ (70 ml). The mixture was then stirred for 1h. 3,5-Dimethylphenol (6.82 g, 55.9 mmol, freshly sublimed) in $Et_2O$ (40 ml) was then slowly added and the mixture stirred for another 1h. The mixture was cooled to -78°C and added slowly to the product of step A (3.04g, 13.63 g) in $Et_2O$ (200 ml), whereby a pale yellow colour appeared. The solution was warmed up to -60°C and stirred for 16h at this temperature. The reaction was quenched with 1N HC1, extracted, dried and evaporated. The majority of the phenol was sublimed off (70°C, 0.1 mm Hg). FC (hexane/$Et_2O$ 10:1 1:1) furnished the title compound (2.73 g, 89%, 81% e.e). After several crystallisations from hexane/$CH_2Cl_2$ (below 90% e.e the racemate crystallises first) the enatiomerically pure sultam was obtained (1.62 g, 53%).

C. Synthesis of N-[Acryloyl] -(3R)-tert-butyl-2,3-dihydro-1,2-benzisothiazole-1,1-dioxide

The product of step B (3.6 g, 16 mmol) was dissolved in DMF (50 ml) and added slowly to NaH (770 mg, 19.2 mmol) in DMF (50 ml). The mixture was stirred for 3h at r.t. and decantated from the remaining precipitate. The solution was then slowly added to a mixture of acryloyl chloride (5.59 g, 64 mmol), copper (1.02 g, 16 mmol) and copper (I) chloride (1.58 g, 16 mmol) in DMF (50 ml) at 0°C. After stirring for another 0.5h at r.t., the mixture was poured into dil. aq. $NaHCO_3/NH_4Cl$. Extraction with $Et_2O$, drying, FC (hexane/EtOAc 4:1) and crystallisation

from hexane/$Et_2O$ gave the pure product (2.74 g, 61%) and recoveed starting material (800 mg, 22%). M.p.: 94-5°C $[a]_D$ = +38.9°, $[a]_{578}$ = +40.4°, $[a]_{546}$ = +46.1°, $[a]_{436}$ = +79.5°, $[a]_{365}$ = +116.9°, (c = 1, $CHCl_3$, T = 25°C).

Example 2

Synthesis of N-[Acryloyl]-(3S) - tert-butyl-2,3 dihydro -1,2-benzisothiazole-1,1-dioxide and Alternative Synthesis of -(3R)- enantiomer

Step A of Example 1 was followed to produce 3-tert-butyl-1,2-benziosothiazole-1,1-dioxide.

B. Synthesis of (3R,S)-tert-butyl-2,3-dihydro-1,2-benzisothiazole 1,1 dioxide

The product of step A (9 g, 40.3 mmol) was dissolved in MeOH (150 ml) and cooled to 0°C. Sodium borohydride (1.5 g, 40.3 mmol) was slowly added in portions. The mixture was stirred for 0.5 h at r.t. and the solvent evaporated. Trituration with EtOAc (150 ml), washing of the organic phase successively with 1N HC1, $H_2O$ and brine, drying ($MgSO_4$) and evaporation gave the crude sultam.

Recrystallisation from hexane/$CH_2Cl_2$ furnished the pure product (8.9 g, 98%).

C. Synthesis of N-[10-Camphersulfonyl] -3-tert-butyl-2,3-dihydro-1,2-benziosothiazole-1,1-dioxide

The racemic product of step B (17.7 g, 78.7 mmol) was dissolved in THF (200 ml) and slowly added to a slurry of NaH (4.72 g, 118 mmol) in THF (200 ml). The mixture was stirred for 3 h and the 10 camphorsulfonic acid chloride (32 g, 118 mmol) added. After stiring for 16 h at r.t. the mixture was slowly poured into sat. aq. $Na_2CO_3$. The solution was extracted several times with $CH_2Cl_2$. The organic phase was dried ($MgSO_4$) and filtered through $SiO_2$. Evaporation furnished the diastereomeric mixture (20.3 g, 54%). Several careful crystallisations from hexane/$CH_2Cl_2$ separated the two diastereoisomers. N-[10-Camphorsulfonyl]-(3S)-tert-butyl-2, 3-dihydro-1,2-benzisothiazole-1,1-dioxide (9.73 g, 26%). M.p.: 258-60°C and N-[10-Camphorsulfonyl]-(3R)-tert-butyl-2,3-dihydro-1,2-benzisothiazole-1,1-dioxide (7.88 g, 21%). M.p.: 197-8°C.

D. Synthesis of (3S)-tert-butyl-2,3-dihydro-1,2-benziosothiazole 1,1 dioxide

The (35) camphor adduct (9.73 g, 20.3 mmol) was mixed with 50 ml conc. $H_2SO_4$ and stirred at r.t. until it appeared as a clear solution (1.5 h). The mixture was cooled (0°C) and slowly diluted with $H_2O$ (200 ml). The solution obtained was poured into sat. aq. $Na_2CO_3$, extracted with $Et_2O$, dried and evaporated. Recrystallisation from hexane/$CH_2Cl_2$ furnished the pure (3S)-sultam (4.4 g, 96%) m.p: 129-30°C $[a]_D$ = 53.9°, $[a]_{578}$ = 56.0°, $[a]_{546}$ = 62.9°, $[a]_{436}$ = 97.8°, $[a]_{365}$ = -130.1°, (c = 1, $CHCl_3$, T = 20°C. The (3R)-derivative was prepared in analogous fashion from the (3R) camphor adduct.

General procedure for carrying out step (1) of the claimed process

N-Chlorosuccinimide (NCS) (3.9 mmol, 5 eg.) was placed in dry $CH_2Cl_2$ (3 ml) and then dry pyridine (0.7 mmol, 0.9 eq.) was added. The relevant oxime (3.9 mmol, 5 eq.) in $CH_2Cl_2$ (1.5 ml) was then added in one portion at r.t.. Formation of the chloroxime was taken as complete when the NCS was fully taken into solution (10 min). The chloroxime was used directly.

To a solution of the product of Example 1 or 2 (0.78 mmol, 1 eq.) in dry toluene (25 ml) at r.t. was added the chloroxime (3.9 mmol, 5 eq.). Dehydrohalogenation was performed slowly in situ by the addition of triethylamine (5.5 mmol, 7 eq.) in toluene (5 ml) over 1-2h (the reaction mixture become yellow and turbid). The reaction was continued at r.t. until the appearance of a more polar product by tlc was complete. Sat. ammonium chloride solution was added, the solution extracted several times with ether, dried and purified.

Example 3

Preparation of N-[(1S)-1-(3-tert-butyl-4,5-dihydro-5-isoxazolyl) carbonyl]-(3S)-tert-butyl-2,3-dihydro-1,2-benzisothiazole 1,1 dioxide

The general procedure was followed using the (3S) enantiomer of Example 2 (218 mg, 0.78 mmol) and the nitrile oxide formed from pivaldehyde oxime in toluene/$CH_2Cl_2$ (6:1, 35 ml). Workup, FC (hexane/EtOAc 4:1) and recrystallisation from hexane/EtOAc (8:1) furnished the pure title compound (253 mg, 86%). M.p.: 135-6°C.

Example 4

Preparation of N-[(1S)-1-(3-methyl-4,5-dihydro-5-isoxazolyl) carbonyl]-(3S)-tert-butyl-2,3-dihydro-1,2-benzisothiazole 1,1 dioxide

The general procedure was followed using the (3S) enantiomer of Example 2 (193 mg, 0.69 mmol) and the nitrile oxide formed from acetaldehyde oxime in toluene/$CH_2Cl_2$ (6:1, 35 ml). Workup, FC (hexane/EtOAc 2:1) and recrystallisation from hexane/EtOAc (8:1) furnished the pure title compound (166 mg, 72%). M.p.: 145-6°C.

Example 5

Preparation of N-[(1S)-1-(3-Ethyl-4,5-dihydro-5-isoxazolyl) carbonyl]-(3S)-tert-butyl-2,3-dihydro-1,2-benzisothiazole 1,1 dioxide

The general procedure was followed using the (3S) enantiomer of Example 2 (213 mg, 0.76 mmol) and the nitrile oxide formed from propionaldehyde oxime in toluene/$CH_2Cl_2$ (6:1, 35 ml). Workup, FC (hexane/EtOAc 2:1) and recrystallisation from hexane/EtOAc (8:1) furnished the pure title compound (166 mg, 72%). M.p.: 139-40°C.

Example 6

Preparation of N-[(1S)-1-(3-Phenyl-4,5-dihydro-5-isoxazolyl) carbonyl]-(3S)-tert-butyl-2,3-dihydro-1,2-benzisothiazole 1,1 dioxide

The general procedure was followed using the (3S) enantiomer of Example 2 (564 mg, 2.02 mmol) and the nitrile oxide formed from benzaldehyde oxime in toluene/$CH_2Cl_2$ (6:1, 70 ml). Workup, FC (hexane/EtOAc 4:1) and recrystallisation from hexane/EtOAc (8:1) furnished the pure title compound (627 mg, 78%). M.p.: 182-3°C.

Example 7

Preparation of N-I[(1R)-1-(3-Phenyl-4,5-dihydro-5-isoxazolyl) carbonyl]-(3R)-tert-butyl-2,3-dihydro-1,2-benzisothiazole 1,1 dioxide

The general procedure was followed using the product of Example 1 (400 mg, 1.43 mmol) and the nitrile oxide formed from benzaldehyde oxime in toluene/$CH_2Cl_2$ (6:1, 70 ml). Workup, FC (hexane/EtOAc 4:1) and recrystallisation from hexane/EtOAc (8:1) furnished the pure title compound (450 mg, 79%). M.p.: 182-3°C.

General procedure for carrying out step (2) of the claimed process

The product of step (1) (1eq.) was dissolved in THF (0.2 M solution) at r.t. and L-selectride (lithium trisecbutylborohydride-2eq) was added. The mixture was stirred at r.t. until completion (16h), quenched with $H_2O$ (2 ml), and then 1 N NaOH and $H_2O_2$ (30%, 3 ml) were added. After 1h at r.t., the mixture was saturated with $K_2CO_3$, extracted with $Et_2O$, dried and purified. The chiral auxiliary was recovered by acidifying the aqueous phase and extraction with $Et_2O$.

Example 8

Preparation of (1S)-3-tert-butyl-4,5-dihydro-5-isoxazolemethanol

Following the general procedure, the product of Example 3 (120 mg, 0.32 mmol) was treated with L-selectride (1.27 ml, 1 M in THF, 1.27 mmol) and stirred for 2 days. Workup and FC (hexane/EtOAc 4:1) furnished the pure title compound (25 mg, 50%) and the chiral sultam (70 mg, 97%). $[a]_D$ = +126.5°, $[a]_{578}$ = +131.8°, $[a]_{546}$ = +149.8°, $[a]_{436}$ = +255.0°, [a]365 = +402.8°, (c = 1, $CHCl_3$, T = 25°C [Lit. : $[a]_D$ = +124° (c = 1, $CHCl_3$, T = 25°C].

Example 9

Preparation of (1S)-3-Methyl-4,5-dihydro-5-isoxazolemethanol

Following the general procedure, the product of Example 4 (377 mg, 1.12 mmol) was treated with L-selectride (2.2 ml, 1 M in THF, 2.2 mmol) and stirred for 16h. Workup and FC ($Et_2O$) and distillation (Kugelrohr, B.p.$_{0.1}$: 70-80°C) furnished the pure title compound (84 mg, 65%) and the chiral sultam (239 mg, 95%. $[a]_D$ = +175.6°, $[a]_{578}$ = +183.1°, $[a]_{546}$ = +207.9°, $[a]_{436}$ = +354.9°, $[a]_{365}$ = +561.1°, (c = 1, $CHCl_3$, T = 25°C [Lit. : $[a]_D$ = 172° (c = 1, $CHCl_3$, T = 25°C].

Example 10

Preparation of (1S)-3-Ethyl-4,5-dihydro-5-isoxazolemethanol

Following the general procedure, the product of Example 5 (360 mg, 1.03 mmol) was treated with L-selectride (2.1 ml, 1 M in THF, 2.1 mmol) and stirred for 16h. Workup, FC ($Et_2O$) and distillation (Kugelrohr, B.p.$_{0.1}$: 75-85°C) furnished the pure title compound (98 mg, 74%) and the chiral sultam (218 mg, 94%). $[a]_D$ = +156.6°, $[a]_{578}$ = +163.4°, $[a]_{546}$ = +185.7°, $[a]_{436}$ = +316.9°, $[a]_{365}$ = +501.5°, (c = 0.8, $CHCl_3$, T = 25°C) [Lit. : $[a]_D$ = +154° (c = 0.7, $CHCl_3$, T = 25°C)].

Example 11

Preparation of (1S)-3-Phenyl-4,5-dihydro-5-isoxazolemethanol

Following the general procedure, the product of Example 6 (396 mg, 0.99 mmol) was treated with L-selectride (4 ml, 1 M in THF, 4 mmol) and stirred for 16h. Workup, FC (pentane/$Et_2O$ 1:1) and recrystallisation (pentane/$Et_2O$) furnished the pure title compound (94 mg, 53%) and the chiral sultam (216 mg, 96%). M.p.: 75.6°C $[a]_D$ = +160.8°, $[a]_{578}$ - +168.4°, $[a]_{546}$ = +193.1°, $[a]_{436}$ = +349.7°, $[a]_{365}$ = +612.9°, (c = 1, $CHCl_3$, T = 25°C) [Lit. $[a]_D$ = +161° (c = 1, $CHCl_3$, T = 25°C)].

Example 12

Preparation of (1R)-3-Phenyl-4,5-dihydro-5-isoxazolemethanol

Following the general procedure, the product of Example 7 (310 mg, 0.78 mmol) was treated with L-selectride (1.6 ml, 1 M in THF, 1.6 mmol) and stirred for 16h. Workup, FC (pentane/$Et_2O$ 1:1) and recrystallisation (pentane/$Et_2O$) furnished the pure title compound (91 mg, 66%) and the chiral sultam (163 mg, 95%) M.p.: 75-6°C. $[a]_D$ = 173.1°, $[a]_{578}$ = +181.2°, $[a]_{546}$ = +207.8°, $[a]_{436}$ = +376.6°, $[a]_{365}$ = +660.3°, (c = 1.1, $CHCl_3$, T = 25°C).

## Claims

1. A process for the preparing a chiral oxazoline having the general formula

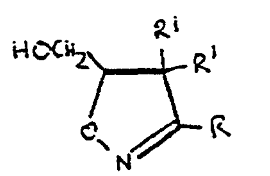

(I)

wherein R is $C_1$ to $C_{10}$ alkyl or phenyl and the $R^1$ groups are independently hydrogen or $C_1$ to $C_4$ alkyl, in substantially optically pure form which process comprises the steps of:

(1) reacting a nitrile oxide of formula R-C=N-O with a single enantiomer of an acryloyl derivative of formula Z-COCH=C($R^1$)$_2$ wherein Z is a moiety having the general formula

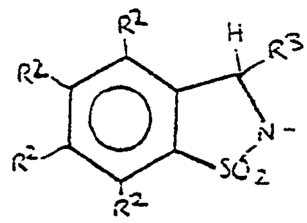

(II)

wherein the $R^2$ groups are independently hydrogen or $C_1$ to $C_{10}$ alkyl and $R^3$ is alkyl or aryl, and (2) reacting the product of step (1) with a hydrogenating agent to produce the oxaline and a chiral sultam of formula ZH.

2. A process as claimed in claim 1 wherein the nitrile oxide in step (1) is generated in situ from the corresponding chloroxime.

3. A process as claimed in claim 1 wherein the product of step (1) is isolated and purified before being used in step (2).

4. A process as claimed in claim 1 wherein the hydrogenating agent used in step (2) is a borohydride derivative of formula $M^+ B(X)3H^-$ where M is an alkali metal and the X groups are independently primary or secondary $C_1$ to $C_{10}$ alkyl.

5. A process as claimed in claim 4 wherein the hydrogenating agent is a salt of the tri-sec-butylborohydride anion.

6. A process as claimed in claim 1 comprising the additional step at (3) recovering the chiral sultam ZH and thereafter treating it with an acryloyl halide of formula YCOCH - $C(R^1)_2$, where Y - C1 or Br, to regenerate the acryloyl derivative of formula Z-COCH=$C(R^1)_2$.

7. A process as claimed in claim 1 wherein the oxazoline produced is one in which the $R^1$ groups are hydrogen.

8. A process as claimed in claim 7 wherein the oxazoline produced is one in which the R group is selected from the group consisting of methyl ethyl, tert-butyl or phenyl.

9. Compounds having the general formula:

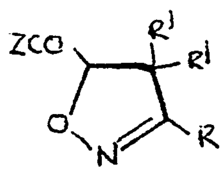

wherein Z, $R^1$ and R are as defined in claim 1.

10. Compounds as claimed in claim 9 in substantially optically pure form.

European Patent Office

# EUROPEAN SEARCH REPORT

Application Number

EP 91 30 3248

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | TETRAHEDRON LETTERS, vol. 29, no. 29, 1988, pages 3555-3558, Oxford, GB; D.P. CURRAN et al.: "The preparation of optically active delta2-isoxazolines. A model for asymmetric induction in the non Lewis acid catalyzed reactions of oppolzer's chiral sultam" * The whole article * --- | 1-8 | C 07 D 261/04<br>C 07 D 417/06 |
| A | JOURNAL OF ORGANIC CHEMISTRY, vol. 52, no. 11, 29th May 1987, pages 2137-2141, Washington, DC, US; D.P. CURRAN et al.: "Asymmetric induction in nitrile oxide cycloadditions to optically active acrylates. Comparisons of acrylate conformations in thermal and acid-catalyzed 1,3-dipolar and Diels-Alder cycloaddition transition states" * The whole article * ----- | 1-8 | TECHNICAL FIELDS SEARCHED (Int. Cl.5)<br>C 07 D 261/00<br>C 07 D 417/00 |

The present search report has been drawn up for all claims

| Place of search | Date of completion of the search | Examiner |
|---|---|---|
| THE HAGUE | 13-06-1991 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)